# EUROPEAN PATENT APPLICATION

(11) **EP 3 342 402 A1**
(43) Date of publication of application: **04.07.2018**
(21) Application number: 17211105.6
(22) Date of filing: 29.12.2017
(51) Int. Cl.: A61K 9/20, A61K 45/06, A61K 9/28, A61K 31/155, A61K 31/4439

(54) **PHARMACEUTICAL COMPOSITIONS OF METFORMIN HYDROCHLORIDE AND PIOGLITAZONE HYDROCHLORIDE**

(30) Priority: 30.12.2016 TR 201620309
(71) Applicant: Sanovel Ilac Sanayi ve Ticaret A.S., 34460 Istanbul (TR)
(72) Inventor: TÜRKYILMAZ, Ali, 34460 Istanbul (TR); PEHLIVAN AKALIN, Nur, 34460 Istanbul (TR); AYHAN, Merve, 34460 Istanbul (TR)
(74) Representative: Sevinç, Erkan

(57) **Abstract**

The present invention relates to pharmaceutical compositions comprising metformin hydrochloride and pioglitazone hydrochloride.

## Description

### Technical Aspect

The present invention relates to pharmaceutical compositions comprising metformin hydrochloride and pioglitazone hydrochloride.

### Background of the Invention

Diabetes mellitus is a group of disorders of carbohydrate metabolism in which the action of insulin is diminished or absent through altered secretion, decreased insulin activity or a combination of both factors.

Metformin is an oral antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 500 to 850 mg. It is used singly or in combination with sulfonylureas, alpha-glucosidase inhibitors, or insulin.

Metformin hydrochloride, with the chemical name 1,1-dimethylbiguanide hydrochloride, has the following chemical structure of Formula I.

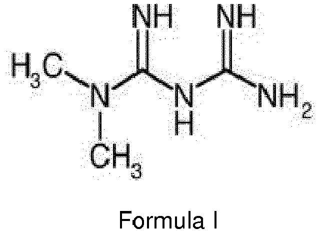

Various patent applications are available in relation to metformin.

Metformin is disclosed with the patent US 3,174,901.

The patent US 6,475,521 discloses a water-soluble pharmaceutical formulation comprising antidiabetics metformin, providing prolonged release.

Pioglitazone is an orally-administered antidiabetics. Pioglitazone belongs to the class of thiazolinediones which act primarily by decreasing insulin resistance. It is not practically soluble in water.

On the other hand, the chemical designation of pioglitazone hydrochloride is (+/-)-5-[[4-[2-(5-ethyl-2-pyridinyl)ethoxy]phenyl]methyl]-2,4-thiazolidinedione hydrochloride, with the following chemical structure of Formula 2.

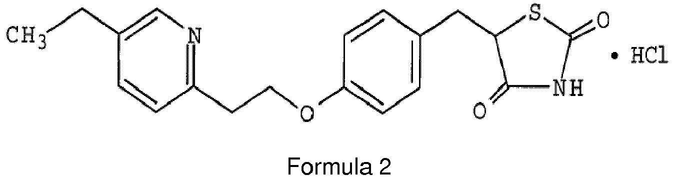

Pioglitazone is disclosed in the patent EP0193256. Various combination formulations have been disclosed, which comprise metformin and pioglitazone.

Combination of pioglitazone hydrochloride and metformin hydrochloride, can more effectively control blood sugar and reduce the incidence rate of adverse reactions. Of the drugs listed by Takeda Pharmaceutical development, the specifications are 15 / 500mg and 15 / 850mg, tradename ACTOPLUSMET.

The patent EP0514452B2 discloses the use of an active agent selected among pioglitazone hydrochloride, metformin, indole amine antidiabetics, thermogenic β-agonists, CS 045, and thiazolidinedione derivatives, in preparing a medicament for use in treating or preventing hypertension in an insulin-resistant patient.

In prior art, metformin hydrochloride has a d (0.5) particle size bigger than 200 µm and this cause the problem of dissolution of metformin hydrochloride, its flowability and its compressibility. Thus, desired amount of metformin hydrochloride in tablet may decrease unexpectedly.

In comparison to prior art, at present invention metformin hydrochloride has a d (0.1) particle size 5 µm to 60 µm, it has a d (0.5) particle size value which is smaller than 115 µm, it has a d (0.9) particle size between 80 µm to 150 µm.

In prior art, pioglitazone hydrochloride has a d (0.5) particle size smaller than 10 µm and this cause the problem of solubility of pioglitazone hydrochloride because of pH of the environment. The solubility of pioglitazone is dependent on pH value.

In this invention, pioglitazone has a d (0.5) particle size between 6 µm to 60 µm so the surface area of pioglitazone is decreased by increasing the particle size and so overcomes the above described problems.

### Description of the Invention

The main object of the present invention is to provide a formulation with high stability, desired level of dissolution rate and bioavailability which overcomes the above described problems in prior art and have additive advantages over them.

The main object of the present invention is to provide an improved pharmaceutical composition, in particular for the treatment of diabetes. The present invention provides a pharmaceutical preparation comprising two active ingredients that act synergistically against a disease or condition.

Another object of the invention is to counteract the contrasting solubility of two active ingredients in a combination drug, and the normal release pattern arising out of the said solubility characteristics, by providing a synergistic formulation wherein the relatively insoluble active ingredient is released immediately and the relatively soluble active ingredient is released in an extended manner.

The main object of the present invention is to provide an improved flowability and compressibility of metformin hydrochloride. It itself flowability and compressibility are poor, the present invention is prepared by first metformin hydrochloride particles to flow and compressibility is improved, it is conducive to the back of the tabletting step of the process. At this invention to overcome this problem particle size of metformin hydrochloride is decreased. Also, a good dissolution profile is provided.

It has surprisingly been found that solid dosage forms of excellent uniformity and showing improved dissolution can be obtained when metformin hydrochloride and pioglitazone hydrochloride are formulated together in wet granulation process. Metformin hydrochloride as the first pharmaceutically active ingredient is granulated together with pioglitazone hydrochloride as second pharmaceutically active ingredient and at least one suitable pharmaceutical excipient. Wet granulation process efficiently counteracts segregation, so it can achieve good dissolution and disintegration properties. At the same time, final dosage forms such as tablets achieve improved hardness and prolonged friability.

The advantages of the present invention are even more significant, as the problem of homogeneity is even more likely to occur when two active substances are incorporated in one final dosage form, especially when two actives are very different regarding the dose and particle size. Improved content uniformity efficiently contributes to a marked increase in bioavailability. Improved content uniformity also favors to avoid toxicity in the otherwise possible event that the amount of drug substance would be too high.

The invention is intended, by combination of a compound having metformin hydrochloride with pioglitazone hydrochloride, to perform especially remarkable effects, to reduce undesirable side effects and to cover up defects observed in administration of a medicine consisting of a single component.

In one embodiment of the invention, a solid oral pharmaceutical composition comprises metformin or a pharmaceutically acceptable salt thereof and pioglitazone or a pharmaceutically acceptable salt thereof.

According to this embodiment, the solid oral pharmaceutical composition comprises metformin hydrochloride and pioglitazone hydrochloride.

According to this embodiment, the total amount of metformin hydrochloride in the composition is 50.0% to 90.0% by weight, preferably 70.0% to 85.0% by weight and the total amount of pioglitazone hydrochloride in the composition is 0,1% to 8.0% by weight, preferably 0.5% to 5.0% by weight.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

In the present invention, metformin hydrochloride has a d (0.1) particle size between 1 µm to 80 µm, preferably it has a d (0.1) particle size between 5 µm to 60 µm and it has a d (0.5) particle size smaller than 115 µm, preferably d (0.5) particle size smaller than 80 µm, more preferably d (0.5) particle size between 15 µm to 50 µm. Also, it has a d (0.9) particle size between 80 µm to 150 µm, preferably it has a d (0.9) particle size between 100 µm to 130 µm.

In the present invention, pioglitazone hydrochloride has a d (0.1) particle size between 1 µm to 10 µm, preferably it has ad (0.1) particle size between 2 µm to 6 µm and it has a d (0.5) particle size between 6 µm to 60 µm, preferably it has a d (0.5) particle size between 8 µm to 40 µm, more preferably d (0.5) particle size between 10 µm to 30 µm. Also, it has a d (0.9) particle size between 50 µm to 90 µm, preferably it has a d (0.9) particle size between 60 µm to 80 µm.

According to one embodiment of the invention, the weight ratio of d (0.5) particle size of metformin hydrochloride to d (0.5) particle size of pioglitazone hydrochloride is 0.01-10.0, preferably the ratio is 0.1-5.0, more preferably the ratio is 0.5-3.0.

According to one embodiment of the invention, the solid oral pharmaceutical composition further comprises at least one pharmaceutically acceptable excipient selected from diluents, disintegrants, binders, lubricants or mixtures thereof.

Suitable diluents are selected from group comprising microcrystalline cellulose, lactose, starch, mannitol, calcium hydrogen phosphate dihydrate, dicalcium hydrogen phosphate anhydrate, calcium phosphate trihydrate, silicium dioxide, glucose or mixtures thereof. Preferably the diluent is microcrystalline cellulose.

Suitable disintegrants are selected from group comprising croscarmellose sodium, microcrystalline cellulose, calcium carboxymethyl cellulose, sodium carboxymethyl cellulose, sodium starch glycollate, pregelatinized starch, alginates or mixtures thereof. Preferably the disintegrant is croscarmellose sodium.

Suitable binders are selected from group comprising polyvinylpyrrolidone, lactose, starch, sugars, guar gum, tragachanti gum, microcrystalline cellulose, methyl cellulose, hydroxylpropyl cellulose, hydroxypropyl methyl cellulose, carboxymethyl cellulose, pectin or mixtures thereof. Preferably the binder is polyvinylpyrrolidone.

Suitable lubricants are selected from group comprising magnesium stearate, stearic acid, calcium stearate, zinc stearate, magnesium oxide, glycols and oils, hydrogenated vegetable oil, corn starch, colloidal silicon dioxide, talc or mixtures thereof. Preferably the lubricant is magnesium stearate.

Solubility is the property of a solid, liquid, or gaseous chemical substance called solute to dissolve in a solid, liquid, or gaseous solvent. The solubility of a substance fundamentally depends on the physical and chemical properties of the solute and solvent as well as on temperature, pressure and the pH of the solution. Some excipients are dissolved in water well and some not.

In one embodiment of the invention, the water soluble expients are selected from polyvinylpyrrolidone, microcrystalline cellulose, starch, mannitol, lactose, silicium dioxide, glucose, guar gum, methanol, pectin, methyl cellulose or mixtures thereof.

In one embodiment of the invention, the water insoluble expients are selected from croscarmellose sodium, magnesium stearate, zinc stearate, talc, colloidal silicon dioxide, glycolys or mixtures thereof.

According to this embodiment, the at least one water soluble excipient comprises 5% to 20% by weight of the total formulation.

According to this embodiment, the at least one water insoluble excipient comprises 1% to 10% by weight of the total formulation.

According to this embodiment, the weight ratio of water soluble excipients to water insoluble excipients is 0.1-10.0, preferably the ratio is 0.5-8.0, more preferably the ratio is 1.0-5.0.

In one embodiment of the invention, the solid oral pharmaceutical composition is in the form of coated tablet, trilayer tablet, bilayer tablet, multilayer tablet, orally disintegrating tablet, mini tablet, pellet, buccal tablet, sublingual tablet, effervescent tablet, immediate release tablet, modified release tablet, film-coated tablet, pill, capsule, oral granule, powder.

According to this embodiment, the solid oral pharmaceutical composition is in the form of a film-coated tablet.

According to this embodiment, the film-coating comprises hydroxypropylmethyl cellulose, titanium dioxide, talc, polyethylene glycol or mixtures thereof.

According to this embodiment, the weight ratio of hydroxypropylmethyl cellulose to titanium dioxide is 0.1-10.0, preferably the ratio is 0.2-6.0.

The tablets are coated with at least one film coating, this enhance in dissolution profile and mechanical stability.

Metformin hydrochloride is an excellent water soluble drug, thus the film coating is preferably formed of permeability, smooth drug release effect so metformin hydrochloride releases to achieve the desired release rate.

Pioglitazone hydrochloride has relatively low solubility in contrast to metformin hydrochloride. In the present invention, a water-soluble polymer is added to the film coating, preferably hydroxypropyl methylcellulose as porogenic agent to form a coating film hydration channels thereby increasing the permeability of the membrane coating, pioglitazone hydrochloride releases pellets to achieve the desired release rate.

In one embodiment of the invention, the composition comprises 50.0-90.0% by weight metformin hydrochloride, 0.1-8.0% by weight of pioglitazone hydrochloride, 5.0-20.0% by weight of microcrystalline cellulose, 1.0-8.0% by weight of croscarmellose sodium, 0.01-5.0% by weight of magnesium stearate, 1.0-8.0% by weight of polyvinylpyrrolidone, 0.5-6.0% by weight of film coating, 1.0-15.0% by weight of ethanol, 1.0-15.0% by weight of pure water.

The compositions of the invention are further characterized by good stability results.

In this present invention, an immediate dissolution of pioglitazone and metformin is obtained and have a good content uniformity and have a simple preparation process, in favor of industrial production.

Another aspect of the present invention is to provide a process for preparing the solid oral pharmaceutical composition comprises the following steps:
a) mixing metformin hydrochloride, pioglitazone hydrochloride and microcrystalline cellulose (Avicel pH 101) for 5 minutes
b) mixing polyvinylpyrrolidone (K30), pure water and ethanol in separete and granulating the mixture
c) drying the granules until 2.0% - 3.0% humidity left
d) sieving the dried granules to have 710 µm particle size
e) adding croscarmellose sodium into the sieved granules and mixing for 15 minutes
f) adding magnesium stearate and mixing the total mixture for 3 minutes
g) compressing the total mixture into tablets
h) preparing an aqueous solution of coating material and coating the tablets with this solution in a way to form a film layer (Opadry White).

### Example 1

This example describes the solid oral pharmaceutical composition comprising metformin hydrochloride and pioglitazone hydrochloride. The active ingredient and excipients of the composition are given below:
The solid oral pharmaceutical composition of metformin hydrochloride and pioglitazone hydrochloride

| | **Amount (% by weight of the total)** |
|---|---|
| Metformin hydrochloride | 73.0-80.0 |
| Pioglitazone hydrochloride | 0.8-3.0 |
| Microcrystalline cellulose | 8.0-15.0 |
| Croscarmellose sodium | 2.0-6.0 |
| Magnesium stearate | 0.08-3.0 |
| Polyvinylpyrrolidone | 3.0-6.0 |
| Film coating | 1.0-4.0 |
| Ethanol* | 3.0-10.0 |
| Pure water* | 3.0-10.0 |

| | |
|---|---|
| * It is removed from the medium during the process | |

| **Coating Material (Opadry White) Ingredients** | **Amount (% by weight of the coating)** |
|---|---|
| Hydroxypropylmethyl cellulose | 60.0-80.0 |
| Titanium dioxide | 10.0-25.0 |
| Talc | 1.0-15.0 |
| Polyethylene glycol | 1.0-7.0 |

A process for preparing the solid oral pharmaceutical composition in example 1 comprises the following steps:
a) mixing metformin hydrochloride, pioglitazone hydrochloride and microcrystalline cellulose (Avicel pH 101) for 5 minutes
b) mixing polyvinylpyrrolidone (K30), pure water and ethanol in separete and granulating the mixture
c) drying the granules until 2.0% - 3.0% humidity left
d) sieving the dried granules to have 710 µm particle size
e) adding croscarmellose sodium into the sieved granules and mixing for 15 minutes
f) adding magnesium stearate and mixing the total mixture for 3 minutes
g) compressing the total mixture into tablets
h) preparing an aqueous solution of coating material and coating the tablets with this solution in a way to form a film layer (Opadry White)

## Claims

1. A solid oral pharmaceutical composition comprising metformin hydrochloride whose total amount in the composition is 50.0% to 90.0% by weight and pioglitazone hydrochloride whose total amount in the composition is 0,1% to 8.0% by weight.

2. The solid oral pharmaceutical composition according to claim 1, wherein metformin hydrochloride whose total amount in the composition is 70.0% to 85.0% by weight and pioglitazone hydrochloride whose total amount in the composition is 0,5% to 5.0% by weight.

3. The solid oral pharmaceutical composition according to claim 2, wherein metformin hydrochloride has a d (0.5) particle size smaller than 115 µm, preferably has a d (0.5) particle size smaller than 80 µm, more preferably d (0.5) particle size beetween 15 µm to 50 µm.

4. The solid oral pharmaceutical composition according to claim 2, wherein pioglitazone hydrochloride has a d (0.5) particle size between 6 µm to 60 µm, preferably it has a d (0.5) particle size between 8 µm to 40 µm, more preferably d (0.5) particle size between 10 µm to 30 µm.

5. The solid oral pharmaceutical composition according to claim 3 and 4, wherein the weight ratio of d (0.5) particle size of metformin hydrochloride to d (0.5) particle size of pioglitazone hydrochloride is 0.01-10.0, preferably the ratio is 0.1-5.0, more preferably the ratio is 0.5-3.0.

6. The solid oral pharmaceutical composition according to claim 2, wherein metformin hydrochloride has a d (0.1) particle size between 1 µm to 80 µm, preferably it has a d (0.1) particle size between 5 µm to 60 µm.

7. The solid oral pharmaceutical composition according to claim 2, wherein metformin hydrochloride has a d (0.9) particle size between 80 µm to 150 µm, preferably it has a d (0.9) particle size between 100 µm to 130 µm.

8. The solid oral pharmaceutical composition according to claim 2, wherein pioglitazone hydrochloride has a d (0.1) particle size between 1 µm to 10 µm, preferably it has a d (0.1) particle size between 2 µm to 6 µm.

9. The solid oral pharmaceutical composition according to claim 2, wherein pioglitazone hydrochloride has a d (0.9) particle size between 50 µm to 90 µm, preferably it has a d (0.9) particle size between 60 µm to 80 µm.

10. The solid oral pharmaceutical composition according to any preceding claims, wherein the composition further comprises at least one pharmaceutically acceptable excipient selected from diluents, disintegrants, binders, lubricants or mixtures thereof.

11. The solid oral pharmaceutical composition according to claim 10, wherein the weight ratio of water soluble excipients to water insoluble excipients is 0.1-10.0, preferably the ratio is 0.5-8.0, more preferably the ratio is 1.0-5.0.

12. The solid oral pharmaceutical composition according to any preceding claims, wherein the composition is in the form of coated tablet, trilayer tablet, bilayer tablet, multilayer tablet, orally disintegrating tablet, mini tablet, pellet, buccal tablet, sublingual tablet, effervescent tablet, immediate release tablet, modified release tablet, film-coated tablet, pill, capsule, oral granule, powder.

13. The solid oral pharmaceutical composition according to claim 12, wherein the composition is in the form of a film-coated tablet.

14. The solid oral pharmaceutical composition according to claim 13, wherein the film-coating comprising hydroxypropylmethyl cellulose, titanium dioxide, talc, polyethylene glycol or mixtures thereof.

15. The solid oral pharmaceutical composition according to claim 14, wherein the weight ratio of hydroxypropylmethyl cellulose to titanium dioxide is 0.1-10.0, preferably the ratio is 0.2-6.0.

16. The solid oral pharmaceutical composition according to any preceding claims, wherein the composition comprises;
• 50.0-90.0% by weight metformin hydrochloride,
• 0.1-8.0% by weight of pioglitazone hydrochloride,
• 5.0-20.0% by weight of microcrystalline cellulose,
• 1.0-8.0% by weight of croscarmellose sodium,
• 0.01-5.0% by weight of magnesium stearate,
• 1.0-8.0% by weight of polyvinylpyrrolidone,
• 0.5-6.0% by weight of film coating,
• 1.0-15.0% by weight of ethanol,
• 1.0-15.0% by weight of pure water.

17. A process for preparing the solid oral pharmaceutical composition according to claim 16, comprising the following steps:
a) mixing metformin hydrochloride, pioglitazone hydrochloride and microcrystalline cellulose for 5 minutes
b) mixing polyvinylpyrrolidone, pure water and ethanol in separete and granulating the mixture
c) drying the granules until 2.0% - 3.0% humidity left
d) sieving the dried granules to have 710 µm particle size
e) adding croscarmellose sodium into the sieved granules and mixing for 15 minutes
f) adding magnesium stearate and mixing the total mixture for 3 minutes
g) compressing the total mixture into tablets
h) preparing an aqueous solution of coating material and coating the tablets with this solution in a way to form a film layer.
